# EUROPÄISCHE PATENTSCHRIFT

(11) **EP 1 441 658 B1**
(45) Veröffentlichungstag und Bekanntmachung des Hinweises auf die Patenterteilung: **08.06.2005**
(21) Anmeldenummer: 02767462.1
(22) Anmeldetag: 31.08.2002
(51) Int. Cl.: A61B 19/02, A61L 2/26

(54) **STERILBEHÄLTER**
STERILE CONTAINER
CONTENANT STERILE

(30) Priorität: 15.11.2001 DE 10156935
(43) Veröffentlichungstag der Anmeldung: 04.08.2004
(73) Patentinhaber: Aesculap AG & Co. KG, 78532 Tuttlingen (DE)
(72) Erfinder: GLEICHAUF, Wilhelm, 78532 Tuttlingen-Möhringen (DE); JAKAB, Mariana, 78532 Tuttlingen (DE); OERTMANN, Friedrich-Wilhelm, 78532 Tuttlingen (DE); RENNER, Torsten, 78532 Tuttlingen (DE); SCHUSTER, Stefan, 78532 Tuttlingen (DE); SCHWANKE, Wolfgang, 78604 Rietheim-Weilheim (DE)
(74) Vertreter: HOEGER, STELLRECHT & PARTNER Patentanwälte
(86) Internationale Anmeldenummer: PCT/EP2002/009749
(87) Internationale Veröffentlichungsnummer: WO 2003/041604

(56) Entgegenhaltungen:
- DE-A- 3 500 026
- DE-U- 8 213 351
- US-A- 4 661 326
- US-A- 6 145 687

## Beschreibung

Die Erfindung betrifft einen Sterilbehälter, insbesondere zur Aufnahme und sterilen Aufbewahrung von chirurgischem Besteck oder Material, umfassend einen durch einen Behälterboden und Behälterwände gebildeten Aufnahmeraum und einen Deckel zum Verschließen des Aufnahmeraums mit einer mit einem Sterilfilter verschließbaren Gasaustauschöffnung, wobei auf einer Außenseite des Deckels mindestens eine von der Gasaustauschöffnung weg weisende, nach außen relativ zu einer Deckelebene abfallende Einströmkante vorgesehen ist und wobei ein das Sterilfilter beabstandet überdeckendes Schutzelement vorgesehen.

Sterilbehälter der eingangs beschriebenen Art werden, nachdem sie beispielsweise mit chirurgischem Besteck und/oder Material gefüllt wurden, in einem Sterilisator mit Dampf oder anderen Medien sterilisiert. Damit taucht regelmäßig das Problem auf, daß der Deckel nach dem Sterilisiervorgang mit Flüssigkeit benetzt ist, insbesondere mit Restkondensat. Dieses kann dann unerwünschterweise auf das Sterilfilter gelangen. Das gleiche Problem tritt auf, wenn der Sterilbehälter aus Versehen mit einer beliebigen Flüssigkeit beaufschlagt wird.

Ein Beispiel für ein Sterilbehälter der eingangs beschriebenen Art ist aus der DE 82 13 351 U1 bekannt, der einen Aufnahmeraum, einen Deckel, eine Gausaustauschöffnung mit Sterilfilter und eine nach außen abfallende Einströmkante auf der Außenseite des Deckels umfaßt.

Es ist Aufgabe der vorliegenden Erfindung, einen Sterilbehälter der eingangs beschriebenen Art derart zu verbessern, daß der Zusammenbau des Sterilbehälters erleichtert und die Wartungsfreundlichkeit erhöht wird.

Diese Aufgabe wird erfindungsgemäß dadurch gelöst, daß mindestens eine Schnappverbindung zum Verbinden des Schutzelements mit dem Deckel vorgesehen.

Durch diese Ausgestaltung, d. h. daß auf der Außenseite des Deckels mindestens eine von der Gasaustauschöffnung weg weisende, nach außen relativ zur Deckelebene abfallende Einströmkante vorgesehen ist, kann Gas oder Dampf entlang der Einströmkante zur Gasaustauschöffnung und durch diese hindurch in den Aufnahmeraum strömen oder umgekehrt. Dagegen kann eine auf den Deckel gelangte Flüssigkeit von der Gasaustauschöffnung weg abfließen. Wasseransammlungen auf dem Deckel werden so vermieden, so daß auch keine Gefahr besteht, daß Flüssigkeit auf das Sterilfilter fließen kann. Das das Sterilfilter beabstandet überdeckende Schutzelement schützt das Sterilfilter vor mechanischer Beanspruchung und vor einer Beaufschlagung mit Flüssigkeit, wobei sich ein konvex vom Deckel weg gewölbtes Schutzelement als vorteilhaft erweist, um Flüssigkeitsansammlungen auf dem Schutzelement zu vermeiden.

Damit die Flüssigkeit sicher und unter allen Bedingungen abfließen kann, ist es vorteilhaft, wenn ein von der Einströmkante und der Dekkelebene eingeschlossener Neigungswinkel mindestens 1° beträgt.

Um ein Abfließen von auf dem Schutzelement befindlicher Flüssigkeit zu erleichtern, ist es günstig, wenn das Schutzelement auf einer Außenseite des Sterilbehälters angeordnet ist.

Gemäß einer bevorzugten Ausführungsform der Erfindung kann vorgesehen sein, daß der Deckel auf seiner Außenseite eine Lagerfläche zur Lagerung des Schutzelements aufweist, die die Gasaustauschöffnung umgibt. Damit läßt sich das Schutzelement definiert am Deckel anbringen oder mit diesem verbinden. Weiterhin fügt es sich vorteilhaft in den Deckel ein, beispielsweise um eine gemeinsame Oberfläche zu bilden.

Um eine möglichst flache Bauform zu gewährleisten, ist es vorteilhaft, wenn die Lagerfläche eine parallel zur Deckelebene verlaufende, in Richtung auf den Aufnahmeraum zurückgesetzte Ringfläche umfaßt.

Damit keine Flüssigkeit in unerwünschter Weise auf das Sterilfilter gelangen kann, ist es von Vorteil, wenn das Schutzelement die Gasaustauschöffnung und die Lagerfläche vollständig überdeckt. Auf dem Schutzelement befindliche Flüssigkeit kann dann von dem Schutzelement abfließen ohne auf die die Gasaustauschöffnung umgebende Lagerfläche oder das die Gasaustauschöffnung bedeckende Sterilfilter zu gelangen.

Vorzugsweise können Auflageflächen zum Stützen des mit dem Deckel verbundenen Schutzelementes vorgesehen sein. Über diese stützt sich das Schutzelement am Deckel ab.

Damit ein definierter Abstand des Schutzelements vom Deckel eingehalten werden kann, umfaßt das Schutzelement vorzugsweise Abstandselemente.

Günstig ist es, wenn das Schutzelement einen Verstärkungsrahmen umfaßt. Insbesondere bei aus weichen oder elastischen Materialien hergestellten Schutzelementen oder auch bei Beaufschlagung mit Heißdampf wird das Schutzelement durch den Verstärkungsrahmen in seiner gewünschten Form stabilisiert.

Besonders vorteilhaft ist es, wenn der Verstärkungsrahmen sich kreuzende, im wesentlichen parallel zur Deckeloberfläche verlaufende Stege umfaßt. Ein solcher Verstärkungsrahmen läßt sich besonders leicht herstellen und bietet bei geringstem Materialbedarf eine ausreichende Stabilität.

Um den konstruktiven Aufwand und die Anzahl der Elemente des Sterilbehälters gering zu halten, ist es günstig, wenn die Abstandshalteelemente durch Vorsprünge und/oder mindestens teilweise durch die Stege gebildet werden. Damit erfüllen die Vorsprünge und/oder die Stege eine Doppelfunktion. Einerseits verstärken sie das Schutzelement, andererseits stellen sie einen gewünschten Abstand des Schutzelements vom Deckel sicher.

Ein weiterer Vorteil ergibt sich, wenn die mindestens eine Schnappverbindung zum Verbinden des Schutzelements mit einem Rastausnehmungen aufweisenden Rand der Gasaustauschöffnung vorgesehen ist. Insbesondere bei nicht rotationssymmetrisch ausgestalteten Schutzelementen kann so eine definierte Positionierung des Schutzelements am Deckel ermöglicht werden. Durch die Schnappverbindung läßt sich das Schutzelement auf einfache Weise mit dem Deckel verbinden und wieder von diesem lösen.

Eine besonders kompakte Bauform des Sterilbehälters ergibt sich, wenn die mindestens eine Schnappverbindung vom Schutzelement in Richtung auf den Deckel hin abstehende, mit einem Rastvorsprung versehene, elastische Federarme umfaßt. Außerdem läßt sich das Schutzelement so leicht am Deckel anclipsen, beispielsweise am Rand der Gasaustauschöffnung.

Das Schutzelement selbst könnte mit Öffnungen versehen sein, um einen Gasaustausch durch das Schutzelement hindurch zur Gasaustauschöffnung mit der Filtereinheit zu ermöglichen. Besonders vorteilhaft ist es jedoch, wenn zwischen dem Schutzelement und dem Deckel mindestens eine mit der Gasaustauschöffnung in Fluidverbindung stehende Gasdurchtrittsöffnung vorgesehen ist. Dadurch wird keine zusätzliche Durchbrechung des Schutzelements notwendig. Die Gasdurchtrittsöffnung wird allein durch die entsprechende Form des Schutzelements und des Deckels gebildet. Auf dem Schutzelement befindliche Flüssigkeit kann ohne weitere Abdeckungen von Durchbrechungen des Schutzelements vollständig vom Schutzelement abfließen.

Um ein Eindringen von Flüssigkeit auf das Sterilfilter zusätzlich zu unterbinden, ist es vorteilhaft, wenn die Gasdurchtrittsöffnung so angeordnet ist, daß eine Gasströmung in einer im wesentlichen quer zur Durchlaßrichtung des Sterilfilters verlaufenden Strömungsrichtung ermöglicht wird.

Eine besonders flache Bauform ergibt sich, wenn die Gasdurchtrittsöffnung eine im Querschnitt doppeltkonvexe Linsenform aufweist. Ferner wird durch die zur Bildung der doppeltkonvexen Linsenform konkav gekrümmte Deckeloberfläche eine Ablaufrinne für Flüssigkeiten gebildet.

Zum Ermöglichen eines Gasaustauschs durch die Gasaustauschöffnung bei gestapelten Sterilbehältern ist es günstig, wenn der Deckel mindestens ein Abstandselement aufweist zum Stapeln eines weiteren Sterilbehälters auf dem Sterilbehälter.

Vorzugsweise ist die Einströmkante zwischen zwei Abstandselementen angeordnet. Dadurch ist eine ausreichende Neigung der Einströmkante möglich, um auf den Deckel gelangte Flüssigkeit abfließen zu lassen.

Damit mehrere Sterilbehälter sicher aufeinander gestapelt werden können, ist es günstig, wenn die Abstandselemente mindestens drei, von der Außenseite des Sterilbehälters weg weisende Vorsprünge umfassen.

Bei einer vorteilhaften Ausführungsform der Erfindung kann vorgesehen sein, daß die Lagerfläche von der mindestens einen Einströmkante und dem mindestens einen Abstandselement begrenzt ist. Dies ermöglicht es, daß Flüssigkeit vom Abstandselement auf die Einströmkante fließt und direkt von dieser abgeleitet wird.

Damit das Schutzelement in die Deckeloberfläche eingepaßt werden kann, ohne daß ein Übergang zwischen dem Schutzelement und dem Deckel freie Kanten sichtbar sind, ist es günstig, wenn die Abstandselemente die Auflageflächen umfassen oder an diese angrenzen.

Der Sterilbehälter ist besonders einfach und kostengünstig herzustellen, wenn der Deckel, das mindestens eine Abstandselement, die Lagerfläche und die mindestens eine Einströmkante einstückig ausgebildet sind.

Um den Aufbau des Sterilbehälters weiter zu vereinfachen ist es von Vorteil, wenn eine das Sterilfilter, einen Träger und ein Halteelement umfassende Filtereinheit vorgesehen ist und wenn das Sterilfilter zwischen dem Träger und dem Halteelement gehalten ist.

Besonders einfach läßt sich die Filtereinheit austauschen, wodurch die Wartungsfreundlichkeit des Sterilbehälters erhöht wird, wenn die Filtereinheit einteilig ausgebildet ist und wenn der Träger, das Sterilfilter und das Halteelement untrennbar miteinander verbunden sind, insbesondere verklebt oder verschweißt.

Bei einer weiteren bevorzugten Ausführungsform der Erfindung kann vorgesehen sein, daß die Filtereinheit bewegbar gelagert ist, daß die Filtereinheit in einer Schließstellung einen Strömungspfad schließt und in einer Durchlaßstellung den Strömungspfad öffnet, so daß ein Gasaustausch in der Schließstellung nur durch das Sterilfilter und in der Durchlaßstellung durch das Sterilfilter und/oder durch den Strömungskanal ermöglicht wird. Dadurch kann die Filtereinheit je nach Bedarf in die Schließstellung oder die Durchlaßstellung überführt werden, je nachdem wie groß ein gewünschter Durchsatz an Gas und/oder Dampf ist.

Um eine Beschädigung des Sterilbehälters zu vermeiden und seinen Aufbau insgesamt zu vereinfachen, ist es vorteilhaft, wenn ein Überdruckventil vorgesehen ist, wenn das Überdruckventil so angeordnet ist, daß es in einer Grundstellung eine Schließstellung einnimmt, wenn es eine Durchlaßstellung einnimmt, wenn ein Druck in einer Umgebung des Sterilbehälters einen Druck im Sterilbehälter um eine vorgegebene Druckdifferenz übersteigt, und wenn die Filtereinheit das Überdruckventil bildet. Die Filtereinheit dient somit einerseits als Filter, andererseits als Überdruckventil. Eine separate Anordnung eines Überdruckventils ist daher nicht mehr erforderlich.

Um die Stabilität des Deckels zu erhöhen, was insbesondere bei großen Gasaustauschöffnungen von Vorteil ist, umfaßt die Gasaustauschöffnung mindestens ein Versteifungselement zum Versteifen des Deckels. Dies kann beispielsweise durch die Gasaustauschöffnung überspannende Stege oder Träger erreicht werden.

Vorzugsweise wird das mindestens eine Verstärkungselement durch einen die Gasaustauschöffnung überdeckenden Steg ausgebildet. Dieser dient gleichzeitig als zusätzlicher Schutz für das Sterilfilter.

Grundsätzlich könnte sich das Versteifungselement an die parallel zur Deckelebene verlaufende, in Richtung auf den Aufnahmeraum zurückgesetzte Lagerfläche anschließen und damit ebenfalls in Richtung auf den Aufnahmeraum zurückgesetzt sein. Günstig ist es jedoch, wenn das mindestens eine Versteifungselement in Richtung vom Aufnahmeraum relativ zur Außenseite des Deckels weg versetzt ist. Dies ermöglicht es dem sich auf dem Versteifungselement bildenden kondensierten Fluid, vom Versteifungselement abzufließen, ohne durch die Gasaustauschöffnung hindurch auf das Sterilfilter zu gelangen.

Um das Sterilfilter noch besser vor dem Eindringen von Fluiden zu schützen, ist bei einer bevorzugten Ausführungsform der Erfindung vorgesehen, daß ein Fluidrückhalteelement zum Verhindern des Einströmens von Fluid von der Außenseite des Deckels durch die Gasaustauschöffnung vorgesehen ist. Dadurch wird verhindert, daß jedwede Form von Fluiden, insbesondere auch Kondensat, auf das Sterilfilter gelangen kann.

Grundsätzlich könnte das Fluidrückhalteelement auf einer Innenseite des Deckels angeordnet sein. Vorteilhaft ist es aber, wenn das Fluidrückhalteelement auf der Außenseite des Deckels angeordnet ist und die Gasaustauschöffnung mindestens abschnittsweise umgibt. Auf diese Weise wird von vornherein verhindert, daß Fluide in den Bereich der Gasaustauschöffnung gelangen können.

Prinzipiell könnte das Fluidrückhalteelement auch durch eine die Gasaustauschöffnung überspannende Membran gebildet werden. Jedoch läßt sich ein verbesserter Gasaustausch dadurch erreichen, daß bei einer bevorzugten Ausführungsform der Erfindung das Fluidrückhalteelement als von der Außenseite des Deckels vorspringender Rand ausgebildet ist. Zudem läßt sich ein solcher Rand besonders einfach und kostengünstig herstellen.

Um Fluide möglichst weit von der Gasaustauschöffnung fern zu halten, ist es günstig, wenn das Fluidrückhalteelement von der Gasaustauschöffnung beabstandet angeordnet ist. Ferner kann der sich an die Gasaustauschöffnung angrenzende und gegen das Eindringen von Fluiden geschützte Bereich beispielsweise als Lagerfläche dienen.

Vorzugsweise überdeckt das Schutzelement die Gasaustauschöffnung und das Fluidrückhalteelement vollständig. Dadurch müssen von dem Schutzelement abfließende Fluide zunächst das Fluidrückhalteelement überwinden, um durch die Gasaustauschöffnung hindurch auf das Sterilfilter zu gelangen.

Besonders einfach und günstig herzustellen ist der Sterilbehälter, wenn der Deckel aus einem Kunststoff hergestellt ist, insbesondere aus Polyetheretherketon (PEEK) oder Polyphenylensulfon (PPSU).

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Gesamtansicht eines Sterilbehälters;
- Figur 2:: eine ausschnittsweise Draufsicht auf einen Sterilbehälter;
- Figur 3:: eine ausschnittsweise Explosionsdarstellung eines Deckels des Sterilbehälters;
- Figur 4:: eine teilweise durchbrochene Querschnittsansicht durch einen Ausschnitt eines Deckels;
- Figur 5:: eine ausschnittsweise, teilweise durchbrochene Seitenansicht eines Deckels;
- Figur 6:: eine vergrößerte perspektivische Teilansicht eines Dekkels;
- Figur 7:: eine Draufsicht auf die Unterseite eines halben Deckels;
- Figur 8:: eine ausschnittsweise Querschnittsansicht einer gasdicht am Deckel gelagerten Filterkassette;
- Figur 9:: eine ausschnittsweise Querschnittsansicht ähnlich Figur 8 mit einer abgehobenen Filterkassette;
- Figur 10:: eine ausschnittsweise Draufsicht auf eine alternative Ausgestaltung eines Deckels; und
- Figur 11:: eine ausschnittsweise Querschnittsansicht 11-11 in Figur 10.

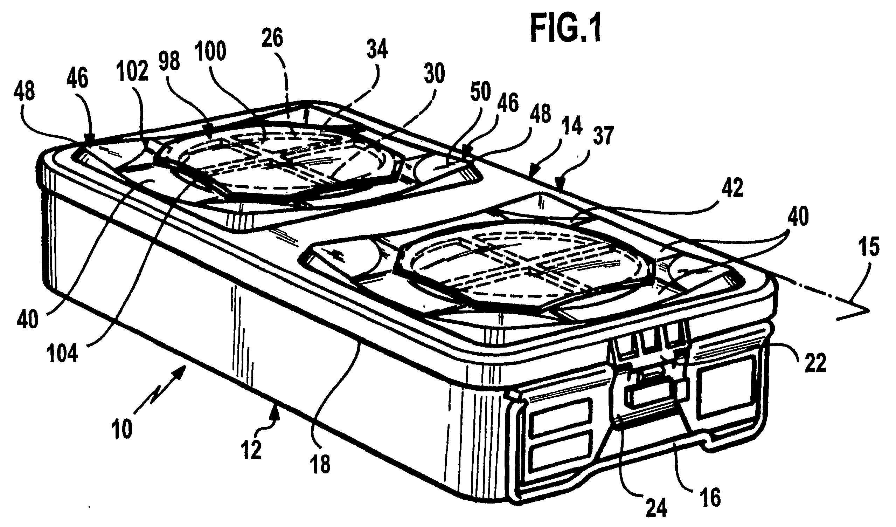

In Figur 1 ist ein insgesamt mit dem Bezugszeichen 10 versehener Sterilbehälter dargestellt, der einen im wesentlichen wannenartigen, quaderförmigen Behälter 12 zum Aufnehmen beispielsweise von chirurgischem Besteck oder Material und einen Deckel 14 umfaßt. An den Stirnflächen des Behälters 12 sind schwenkbar gelagerte Tragegriffe 16 angeordnet.

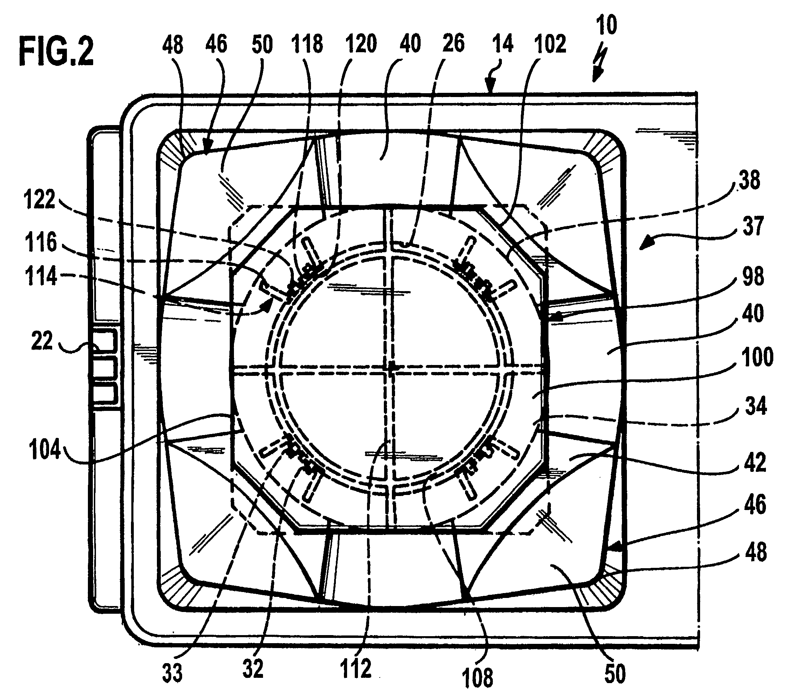

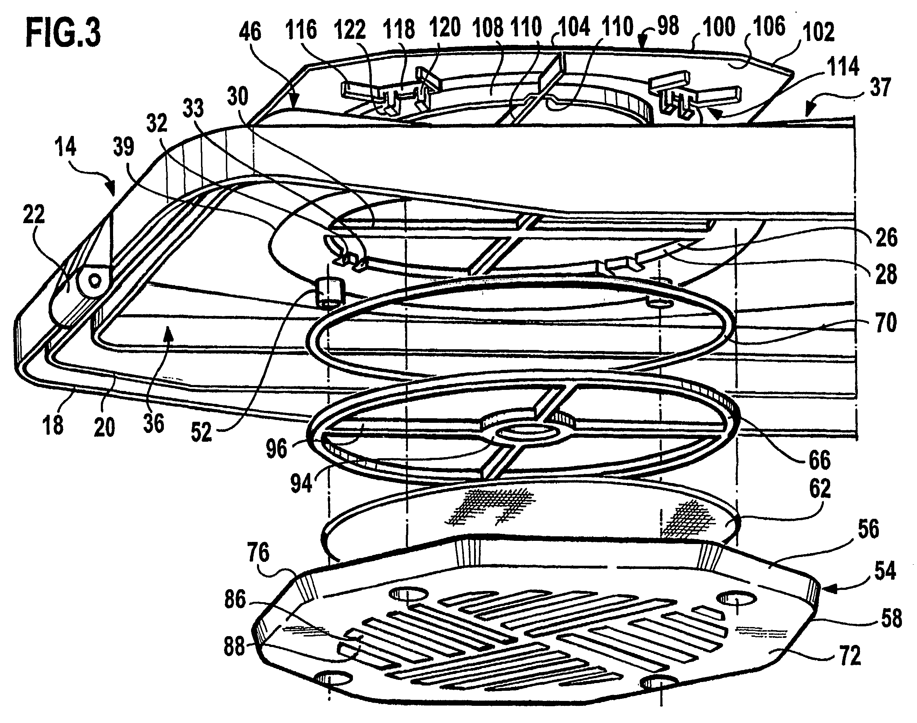

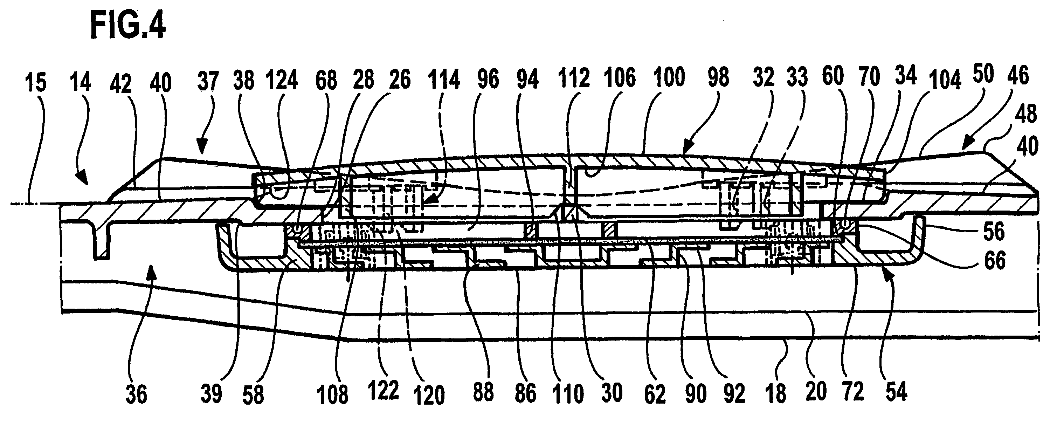

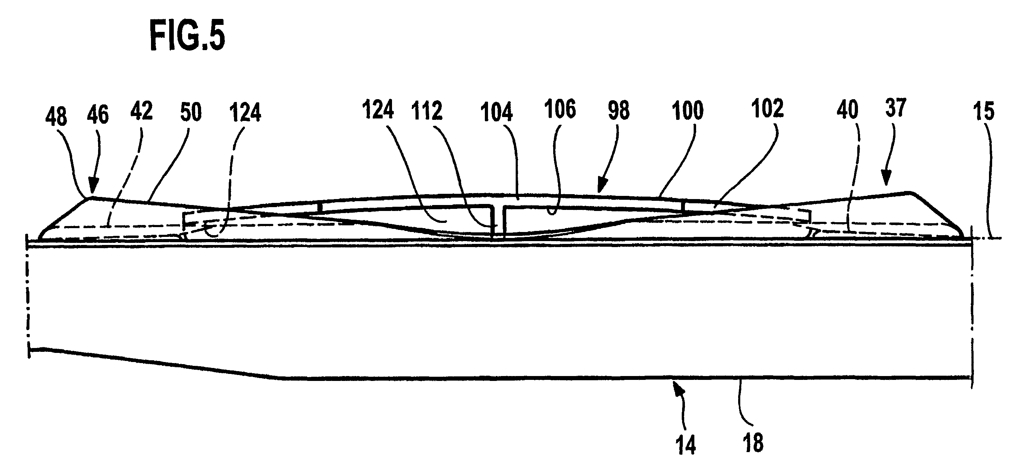

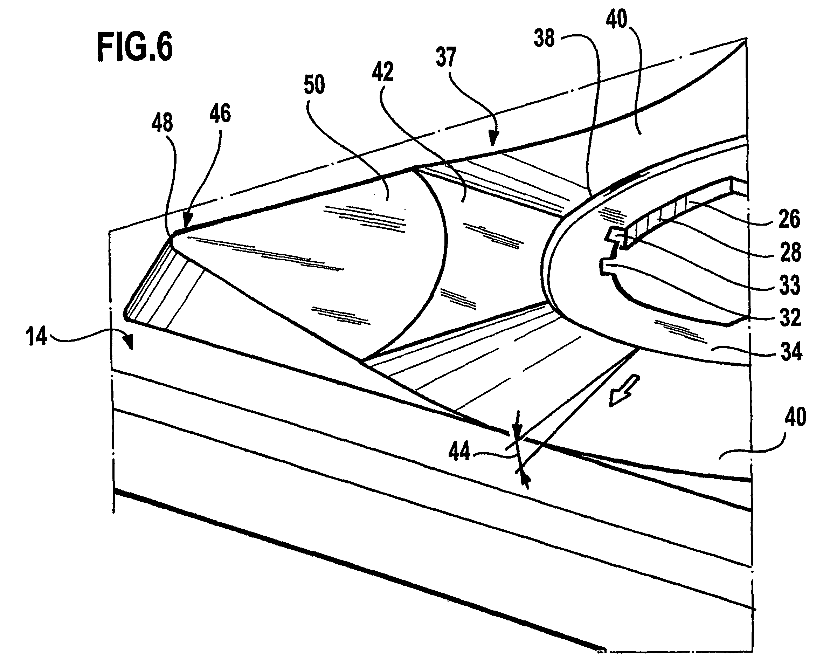

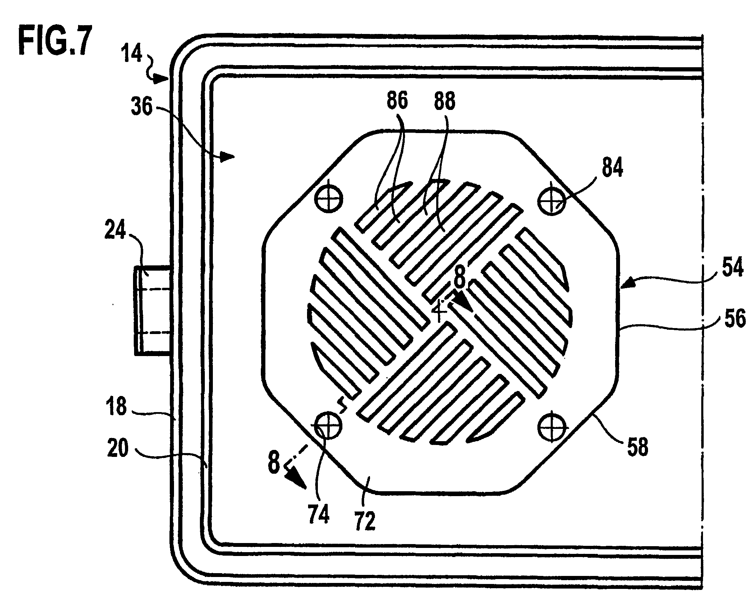

Der Aufbau des Deckels 14 wird anhand der Figuren 2 bis 9 näher erläutert.

Der Deckel 14 ist mit einem umlaufenden, von einer Deckelebene 15 senkrecht abstehenden Rand 18 versehen, so daß der Deckel 14 den Behälter 12 vollständig bedeckt und teilweise umgreift. Parallel zum Rand 18 verläuft beabstandet ein Dichtrand 20, zwischen welchem und dem Rand 18 eine nicht dargestellte Dichtung eingesetzt werden kann. Der Dichtrand 20 befindet sich bei aufgesetztem Deckel 14 innerhalb des Behälters 12. Mit der Dichtung läßt sich eine gasdichte Verbindung zwischen dem Deckel 14 und dem Behälter 12 herstellen.

An seinen schmalen Stirnseiten weist der Deckel 14 jeweils einen Lagerbock 22 auf, an dem eine federnde Verschlußklappe 24 um eine Schwenkachse verschwenkbar ist, die parallel zu einer Schwenkachse des Tragegriffs 16 verläuft, wenn der Deckel 14 auf dem Behälter 12 sitzt.

Der Deckel 14 weist zwei symmetrisch angeordnete kreisförmige Öffnungen 26 auf, die einen parallel zur Deckelebene 15 weisenden Öffnungsrand 28 aufweisen, an dem vier, senkrecht aufeinander zulaufende, sich im Zentrum der Öffnung 26 treffende Stege 30 zur Versteifung des Deckels symmetrisch angeordnet sind. Jeweils zwischen zwei am Öffnungsrand 28 angeordneten Stegen 30 sind symmetrisch zwei Rücksprünge 32 und 33 in den Öffnungsrand 28 eingeschnitten.

Die Öffnung 26 wird von einer Ringfläche 34 umgeben, die parallel zur Deckelebene 15 verläuft, jedoch in Richtung auf eine Deckelunterseite 36 zurückgesetzt ist. Dadurch wird auf einer Deckeloberseite 37 eine innenliegende Ringkante 38, auf der Deckelunterseite 36 eine außenliegende Ringkante 39 gebildet. An die Ringkante 38 grenzen abwechselnd jeweils vier Einströmkanten 40 und vier Auflageflächen 42 an. Die Einströmkanten 40 sind von der Deckeloberseite 37 weg weisend konkav gekrümmt und nach außen geneigt, so daß eine schiefe Ebene mit einem Neigungswinkel 44 zwischen der Einströmkante 40 und der Deckelebene 15 von etwa 2° gebildet wird.

Die Auflageflächen 42 verlaufen parallel zur Deckelebene 15 und weisen in Aufsicht in etwa die Form eines Trapezes auf, jedoch jeweils mit konkav gekrümmten Basiskanten. An die längere der beiden Basislinien der Auflagefläche 42 schließt sich eine dreiseitige Pyramide 46 an, die eine im wesentlichen die Form eines gleichschenkligen rechtwinkligen Dreiecks aufweisende Grundfläche hat. Eine Spitze 48 der Pyramide liegt nahe über den sich rechtwinklig schneidenden Grundseiten der Grundfläche, etwas in Richtung auf die Öffnung 26 hin versetzt.

Dadurch wird eine in etwa die Form eines gleichschenkligen, rechtwinkligen Dreiecks aufweisende Ablauffläche 50 gebildet, die in Richtung auf die Auflagefläche 42 hin geneigt ist. Die Auflagefläche 42 ist parallel zur Deckelebene 15 von der Deckeloberseite 37 weg versetzt.

Insgesamt entsteht somit ein in etwa quadratischer Aufbau auf dem Deckel 14, der gebildet wird durch vier Pyramiden 46, die jeweils in den Ecken des Quadrates sitzen, wobei die Seitenflächen des Quadrats parallel zu Seiten des Deckels 14 verlaufen. Die Einströmkanten 40 sind somit senkrecht zu den Seiten des Deckels 14 hin geneigt. Der Aufbau weist insgesamt zwei parallel zu den Seiten des Deckels 14 verlaufende Symmetrieebenen auf. Die Einströmkante 40 reicht auf ihrer von der Öffnung 26 weg weisenden Seite bis auf das Niveau der Deckelebene 15 herab. In einer seitlichen Ansicht des Deckels ergibt sich somit in etwa eine konkave, von der Deckeloberseite 37 weg weisende Form von der Spitze 48 der Pyramide 46 in einer Ecke des quadratischen Aufbaus bis zur Spitze 48 der Pyramide 46 in einer benachbarten Ecke des Aufbaus.

Benachbart zu den Rücksprüngen 32 und 33 sind insgesamt vier hohlzylindrische, von der Deckelunterseite 36 abstehende, offene Hülsen 52 angeordnet. An diesen ist eine Filterkassette 54 federnd gelagert. Sie umfaßt einen in etwa achteckigen, mit einem Trägerrand 56 versehenen Filterhalter 58 auf, der eine ringförmige Filteraufnahme 60 zum Aufnehmen eines kreisrunden Dauerfilters 62 aufweist. Ein ringförmiger, im Durchmesser größer als die Filteraufnahme 60 ausgestalteter Rücksprung 64 dient zur Aufnahme eines Halteringes 66, der im Querschnitt ein L-förmiges Profil aufweist, dessen einer Schenkel den Dauerfilter 62 in die Fitteraufnahme 60 drückt und dessen anderer Schenkel an dem Rücksprung 64 anliegt. Der Haltering 66 ist mit einer ringförmigen, im Querschnitt etwas mehr als die Hälfte eines Kreises aufweisenden Ringnut zur Aufnahme eines Dichtringes 70 versehen.

Von einem Fiterhalterboden 72 stehen korrespondierend zu den am Deckel 14 angeordneten Hülsen 52 vier Lagerhülsen 74 in Richtung auf den Deckel ab, die vom Filterhalterboden 72 her offen sind und deren andere verschlossene Stirnfläche 76 mit einer zentralen, im Durchmesser zur Hülse 52 korrespondierenden Durchtrittsöffnung 78 versehen ist. Zur Lagerung der Filterkassette 54 werden die vier Lagerhülsen 74 über die Hülsen 52 geschoben, jeweils eine Spiralfeder 80 über die Hülsen 52 gesteckt und anschließend die Lagerhülsen 74 des Filterhalterbodens 72 mit einem Stopfen 82 verschlossen, der einen scheibenförmigen, im Außendurchmesser an den Innendurchmesser der Lagerhülsen 74 angepaßten Kopf 84 aufweist. Indem sich die Spiralfeder 80 am Kopf 84 des Stopfens 82 abstützt, drückt sie die Stirnfläche 76 gegen die Deckelunterseite 36.

Der Filterhalterboden 72 ist insgesamt mit zwanzig unterschiedlich langen streifenförmigen Schlitzen 86 versehen, wobei jeweils fünf Schlitze parallel zueinander verlaufen und in etwa die Fläche eines Viertelkreises bedecken. Schlitze 86 benachbarter Viertelkreise stehen jeweils senkrecht zueinander. Zwischen jeweils zwei Schlitzen 86 wird jeweils ein Schutzsteg 88 gebildet. An jedem Schutzsteg 88 ist eine vom Filterhalterboden 72 weg weisende Leiste 90 angeordnet, an der wiederum parallel zum Filterhalterboden 72 verlaufend ein vom zugehörigen Schutzsteg 88 weg weisender Trägersteg 92 absteht, der jeweils einen Schlitz 86 vollständig überdeckt. Alle Trägerstege 92 bilden eine gemeinsame Ebene, auf der der Dauerfilter 62 aufliegt. Insgesamt bilden die Filteraufnahme 60 sowie die Trägerstege 92 eine Trägerauflage zum Lagern des Dauerfilters 62.

Der Filterhalter 58, der Dauerfilter 62 und der Haltering 66 sind fest miteinander verbunden, beispielsweise geklebt oder geschweißt. Im montierten Zustand drücken die Spiralfedern 88 die Filterkassette 54 gegen die Deckelunterseite 36, wobei mittels des Dichtringes 70 die Filterkassette 54 an der Deckelunterseite 36 abgedichtet wird.

Der Haltering 66 weist einen äußeren und einen konzentrisch dazu verlaufenden inneren Ring 94 auf, von dem radial und symmetrisch angeordnet vier Halteringstege 96 den Haltering 66 stabilisieren.

Durch die versetzte Anordnung von Trägersteg 92 und Schutzsteg 88 ist der auf den Trägerstegen 92 gelagerte Dauerfilter 62 gegen eine direkte mechanische Verletzung geschützt. Auf seiner in Richtung der Öffnung 26 hin zugewandten Seite ist der Dauerfilter 62 jedoch praktisch ungeschützt. Als Schutzelement ist hierfür ein spiegelsymmetrisch ausgebildeter Schutzdeckel 98 vorgesehen, der eine von der Deckeloberseite 37 weg konvex gekrümmte Deckelfläche 100 aufweist, die in Draufsicht die Form eines ungleichseitigen Achtecks mit kurzen Seiten 102 und langen Seiten 104 hat.

Von einer Schutzdeckelunterseite 106 steht eine Ringleiste 108 ab, die vier jeweils paarweise diametral angeordnete Aussparungen 110 umfaßt. Diese sind derart geformt, daß sie die Stege 30 übergreifen können und der Schutzdeckel 98 auf den Stegen 30 aufliegt. Als zusätzliche Abstandshalter sind zwei sich kreuzende Abstandsleisten 112 an der Schutzdeckelunterseite 106 angeordnet, die im Bereich der Aussparungen 110 über die Ringleiste 108 nach außen bis zu den langen Seiten 104 vorstehen. Diese Leisten liegen bei aufgesetztem Schutzdeckel 98 auf der Ringfläche 34 auf.

Zum Befestigen des Schutzdeckels 98 sind vier Rastverbinder 114 vorgesehen, die jeweils aus einem aus drei rechtwinklig zueinander angeordneten, einen U-förmigen Rahmen 116 bildenden Schenkeln bestehen, von denen zwei radial von der Ringleiste 108 nach außen abstehen. Sie bilden, wie freie Enden der Abstandsleisten 112, auf der Ringfläche 34 aufliegende Abstandshalter. Von einem Quersteg 118 des Rahmens 116 stehen parallel zwei Rastarme 120 senkrecht von der Schutzdeckelunterseite 106 ab, die an ihren freien Enden mit einer rechtwinklig abstehenden Rastnase 122 versehen sind. Die vier Rastverbinder 114 sind paarweise diametral gegenüberliegend am Schutzdeckel 98 angeordnet.

Zum Befestigen des Schutzdeckels 98 wird dieser senkrecht in Richtung auf die Deckeloberseite 37 hin bewegt und so orientiert, daß die Aussparungen 110 jeweils auf einen Steg 30 hin weisen und die Rastarme 120 jeweils in einen der beiden Rücksprünge 32 und 33 eingleiten. Sobald die Aussparungen 110 auf den Stegen 30, die Abschlußleisten 112 und die Rahmen 116 auf der Ringfläche 34 aufliegen, verrasten die Rastarme 120 mit dem Öffnungsrand 28, indem die Rastnasen 122 den Öffnungsrand 28 hintergreifen.

Die Größe des Schutzdeckels 98 ist so gewählt, daß im eingesetzten Zustand die Ringfläche 34 vollständig überdeckt ist. Die kurzen Seiten 102 liegen dann auf den Auflageflächen 42 auf, die langen Seiten 104 verlaufen parallel zu Seitenkanten des Deckels 14 und überdecken jeweils teilweise die Einströmkanten 40. Bei eingesetztem Schutzdeckel 98 ist der in der Filterkassette 54 gehaltene Dauerfilter 62 auch von seiner anderen Seite her vollständig gegen eine mechanische Zerstörung geschützt.

Bei einer alternativen, in Figur 2 strichpunktiert angedeuteten Variante eines Deckels sind die langen Seiten 104 des Schutzdeckels 98 verlängert. Die Pyramide 46 weist dann in ihrer Ablauffläche 50 eine zusätzliche Ausnehmung auf, in der die kurze Seite 102 des Schutzdeckels 98 eingefügt ist. Auf diese Weise entsteht dann ein nahtloser Übergang zwischen der Ablauffläche 50 und der Deckelfläche 100.

Der bei eingesetztem Schutzdeckel 98 höchste Punkt desselben liegt unterhalb des höchsten Punkts der vier Pyramiden 46, so daß bei aufeinander gestapelten Sterilbehältern 10 der auf dem Deckel 14 gelagerte Behälter 12 nur auf den Spitzen 48 der Pyramiden 46 aufliegt, die Schutzdeckel 98 jedoch nicht berührt.

Trifft bei einem im wesentlichen horizontal gelagerten Sterilbehälter 10 Flüssigkeit auf die Deckeloberseite 37, beispielsweise auf den Schutzdeckel 98, so kann diese aufgrund der Wölbung des Schutzdeckels 98 zu den kurzen bzw. langen Seiten 102 bzw. 104 abfließen. Von den langen Seiten 104 gelangt die Flüssigkeit auf die Einströmkanten 40, von denen die Flüssigkeit aufgrund von deren Neigung zu den Seiten des Deckels 14 abfließen kann.

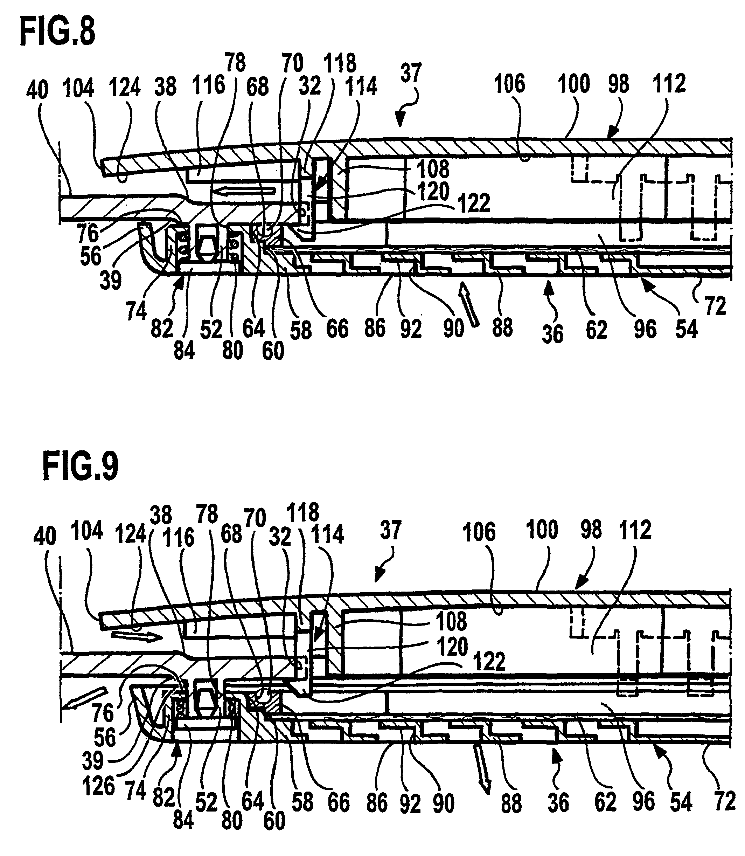

Bei aufgesetztem Schutzdeckel 98 wird eine in einer Seitenansicht im wesentlichen eine doppeltkonvex linsenförmige Einströmöffnung 124 gebildet, durch die ein gasförmiges Fluid ein- und ausströmen kann. Das Fluid strömt dann im wesentlichen parallel zur Deckelebene 15, durch die Öffnung 26 im wesentlichen senkrecht dazu. In einer in Figur 8 dargestellten Grundstellung liegt die Filterkassette 54 mittels des Dichtringes 70 abgedichtet an der Deckelunterseite 36 an. Wird der Sterilbehälter 10 mit Heißdampf beaufschlagt, dann strömt dieser wie vorgenannt beschrieben bis an den Dauerfilter 62 heran. Er tritt durch diesen durch und folgt einem durch den Trägersteg 92, die Leiste 90 und den Schutzsteg 88 gebildeten Strömungskanal ins Innere des Behälters 12.

Übersteigt ein von außen auf den Sterilbehälter 10 einwirkender Druck eine mittels den Spiralfedern 80 einstellbare Kraft, dann wirkt die federnd am Deckel 14 gelagerte Filterkassette 54 als Überdruckventil. Die Filterkassette 54 hebt von der Deckelunterseite 36 ab und öffnet einen in der Grundstellung verschlossenen Strömungskanal 126, wie in Figur 9 dargestellt. Heißdampf kann jetzt nicht nur durch das Dauerfilter 62 hindurchtreten, sondern auch über den Strömungskanal 126 ins Innere des Behälters 12 gelangen. Sobald sich der Druckgradient wieder verringert, drücken die Spiralfedern 80 die Filterkassette 54 an die Deckelunterseite zurück, so daß Ein- und Ausströmen von Dampf oder Gas nur über den Dauerfilter 62 möglich ist. In den Figuren 8 und 9 ist das Aus- bzw. Einströmen eines Gases oder Dampfes anhand der Pfeile angedeutet.

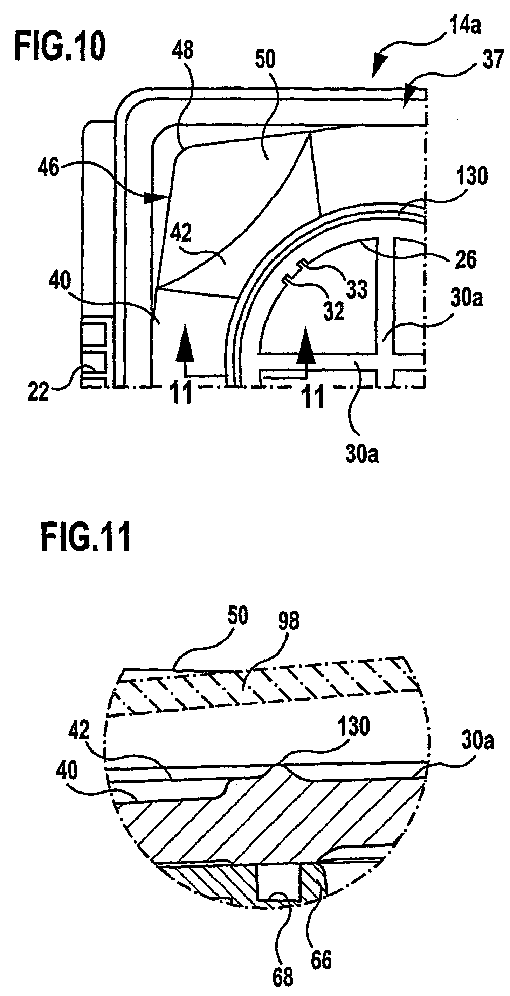

Mit Bezug auf die Figuren 10 und 11 wird nachfolgend eine geringfügig modifizierte Variante des Deckels 14a näher erläutert.

Als zusätzliches Element kann bei einem Deckel 14, wie er im Zusammenhang mit den Figuren 1 bis 9 erläutert wurde, ein Fluidrückhalteelement in Form einer umlaufenden Kante 130 vorgesehen sein. Die umlaufende Kante 130 ist die Öffnung 26 umgebend auf der Deckeloberseite 37 als ringförmiger, von der Deckeloberseite 37 abstehender Vorsprung gebildet. Die Kante schließt sich beim dargestellten Ausführungsbeispiel mit einem geringfügigen Abstand an die Ringkante 38 nach außen in Richtung auf die Einströmkante 40 an. Alternativ wäre es jedoch auch denkbar, daß sich die Kante 130 direkt an den Öffnungsrand 28 oder auch an die Ringkante 38 anschließt. Bei nicht kreisförmig ausgebildeten Gasaustauschöffnungen im Deckel 14 folgt die Kante 130 vorzugsweise der Kontur der Öffnung. Der Schutzdeckel 98 ragt mit seiner langen Seite 104 über die Kante 130 vor, so daß vom Schutzdeckel 98 ablaufende Flüssigkeit direkt auf die Einströmkante 40 abtropft, jedoch nicht über die eine Barriere bildende Kante 130 durch die Öffnung 26 auf das Dauerfilter 62 gelangen kann. Ferner sind die Stege 30a von der Öffnung 26 und der Deckeloberseite 37 weg versetzt, so daß, anders als bei dem im Zusammenhang mit den Figuren 1 bis 9 beschriebenen Deckel 14, keine zurückgesetzte Ringfläche 34 gebildet wird.

Der Behälter 12 kann wahlweise aus Metall oder Kunststoff hergestellt sein. Der Deckel 14 ist vorzugsweise vollständig aus einem Kunststoff hergestellt, beispielsweise aus Polyetheretherketon oder Polyphenylensulfon. Der Dauerfilter ist vorzugsweise aus Polytetrafluorethylen hergestellt.

## Patentansprüche

1. Sterilbehälter (10), insbesondere zur Aufnahme und sterilen Aufbewahrung von chirurgischem Besteck oder Material, umfassend einen durch einen Behälterboden und Behälterwände gebildeten Aufnahmeraum und einen Deckel (14) zum Verschließen des Aufnahmeraums mit einer mit einem Sterilfilter (62) verschlossenen Gasaustauschöffnung (26), wobei auf einer Außenseite (37) des Deckels (14) mindestens eine von der Gasaustauschöffnung (26) weg weisende, nach außen relativ zu einer Deckelebene (15) abfallende Einströmkante (40) vorgesehen ist und wobei ein das Sterilfilter (62) beabstandet überdeckendes Schutzelement (98) vorgesehen ist, **dadurch gekennzeichnet, daß** mindestens eine Schnappverbindung (120, 122) zum Verbinden des Schutzelements (98) mit dem Deckel (14) vorgesehen ist.

2. Sterilbehälter nach Anspruch 1, **dadurch gekennzeichnet, daß** ein von der Einströmkante (40) und der Deckelebene (15) eingeschlossener Neigungswinkel (44) mindestens 1 Grad beträgt.

3. Sterilbehälter nach einem der voranstehenden Ansprüche, **dadurch gekennzeichnet, daß** das Schutzelement (98) auf der Außenseite (37) des Sterilbehälters (10) angeordnet ist.

4. Sterilbehälter nach einem der voranstehenden Ansprüche, **dadurch gekennzeichnet, daß** der Deckel (14) auf seiner Außenseite (37) eine Lagerfläche (34) zur Lagerung des Schutzelementes (98) aufweist, die die Gasaustauschöffnung (26) umgibt.

5. Sterilbehälter nach Anspruch 4, **dadurch gekennzeichnet, daß** die Lagerfläche eine parallel zur Deckelebene (15) verlaufende, in Richtung auf den Aufnahmeraum zurückgesetzte Ringfläche (34) umfaßt.

6. Sterilbehälter nach einem der Ansprüche 4 oder 5, **dadurch gekennzeichnet, daß** das Schutzelement (98) die Gasaustauschöffnung (26) und die Lagerfläche (34) vollständig überdeckt.

7. Sterilbehälter nach einem der voranstehenden Ansprüche, **dadurch gekennzeichnet, daß** Auflageflächen (42) vorgesehen sind zum Stützen des mit dem Deckel (14) verbundenen Schutzelementes (98).

8. Sterilbehälter nach einem der voranstehenden Ansprüche, **dadurch gekennzeichnet, daß** das Schutzelement (98) Abstandshalteelemente (112, 116) zum Halten eines Abstands des Schutzelements (98) vom Deckel (14) umfaßt.

9. Sterilbehälter nach einem der voranstehenden Ansprüche, **dadurch gekennzeichnet, daß** das Schutzelement (98) einen Verstärkungsrahmen (108, 112) umfaßt.

10. Sterilbehälter nach Anspruch 9, **dadurch gekennzeichnet, daß** der Verstärkungsrahmen sich kreuzende, im wesentlichen parallel zur Deckelebene (15) verlaufende Stege (112) umfaßt.

11. Sterilbehälter nach Anspruch 10, **dadurch gekennzeichnet, daß** die Abstandshalteelemente durch Vorsprünge (116) und/oder mindestens teilweise durch die Stege (112) gebildet werden.

12. Sterilbehälter nach einem der voranstehenden Ansprüche, **dadurch gekennzeichnet, daß** die mindestens eine Schnappverbindung (120, 122) zum Verbinden des Schutzelements (98) mit einem Rastausnehmungen (32, 33) aufweisenden Rand (28) der Gasaustauschöffnung (26) vorgesehen ist.

13. Sterilbehälter nach einem der voranstehenden Ansprüche, **dadurch gekennzeichnet, daß** die mindestens eine Schnappverbindung vom Schutzelement (98) in Richtung auf den Deckel (14) hin abstehende, mit einem Rastvorsprung (122) versehene elastische Federarme (120) umfaßt.

14. Sterilbehälter nach einem der voranstehenden Ansprüche, **dadurch gekennzeichnet, daß** zwischen dem Schutzelement (98) und dem Deckel (14) mindestens eine mit der Gasaustauschöffnung (26) in Fluidverbindung stehende Gasdurchtrittsöffnung (124) vorgesehen ist.

15. Sterilbehälter nach Anspruch 14, **dadurch gekennzeichnet, daß** die Gasdurchtrittsöffnung (124) so angeordnet ist, daß eine Gasströmung in einer im wesentlichen quer zur Durchlaßrichtung des Sterilfilters (62) verlaufenden Strömungsrichtung ermöglicht wird.

16. Sterilbehälter nach einem der Ansprüche 14 oder 15, **dadurch gekennzeichnet, daß** die Gasdurchtrittsöffnung (124) eine im Querschnitt doppelkonvexe Linsenform aufweist.

17. Sterilbehälter nach einem der voranstehenden Ansprüche, **dadurch gekennzeichnet, daß** der Deckel (14) mindestens ein Abstandselement (46) aufweist zum Stapeln eines weiteren Sterilbehälters auf dem Sterilbehälter (10), so daß ein Gasaustausch durch die Gasaustauschöffnung (26) bei gestapelten Sterilbehältern (10) möglich ist.

18. Sterilbehälter nach Anspruch 17, **dadurch gekennzeichnet, daß** die Einströmkante (40) zwischen zwei Abstandselementen (46) angeordnet ist.

19. Sterilbehälter nach einem der Ansprüche 17 oder 18, **dadurch gekennzeichnet, daß** die Abstandselemente mindestens drei, von der Außenseite (37) des Sterilbehälters (10) weg weisende Vorsprünge (46) umfassen.

20. Sterilbehälter nach einem der Ansprüche 17 bis 19, **dadurch gekennzeichnet, daß** die Lagerfläche (34) von der mindestens einen Einströmkante (40) und dem mindestens einen Abstandselement (46) begrenzt ist.

21. Sterilbehälter nach einem der Ansprüche 17 bis 20, **dadurch gekennzeichnet, daß** die Abstandselemente (46) die Auflageflächen (42) umfassen oder an diese angrenzen.

22. Sterilbehälter nach einem der Ansprüche 17 bis 21, **dadurch gekennzeichnet, daß** der Deckel (14), das mindestens eine Abstandselement (46), die Lagerfläche (34) und die mindestens eine Einströmkante (40) einstückig ausgebildet sind.

23. Sterilbehälter nach einem der voranstehenden Ansprüche, **dadurch gekennzeichnet, daß** eine das Sterilfilter (62), einen Träger (58) und ein Halteelement (66) umfassende Filtereinheit (54) vorgesehen ist und daß das Sterilfilter (62) zwischen dem Träger (58) und dem Halteelement (66) gehalten ist.

24. Sterilbehälter nach Anspruch 23, **dadurch gekennzeichnet, daß** die Filtereinheit (54) einteilig ausgebildet ist und daß der Träger (58), das Sterilfilter (62) und das Halteelement (66) untrennbar miteinander verbunden sind.

25. Sterilbehälter nach einem der Ansprüche 23 oder 24, **dadurch gekennzeichnet, daß** die Filtereinheit (54) bewegbar gelagert ist, daß die Filtereinheit (54) in einer Schließstellung einen Strömungspfad (126) schließt und in einer Durchlaßstellung den Strömungspfad (126) öffnet, so daß ein Gasaustausch in der Schließstellung nur durch das Sterilfilter (62) und in der Durchlaßstellung durch das Sterilfilter (62) und/oder durch den Strömungspfad (126) ermöglicht wird.

26. Sterilbehälter nach einem der Ansprüche 23 bis 25, **dadurch gekennzeichnet, daß** ein Überdruckventil (54, 52, 80) vorgesehen ist, daß das Überdruckventil (54, 52, 80) so angeordnet ist, daß es in einer Grundstellung eine Schließstellung einnimmt, daß es eine Durchlaßstellung einnimmt, wenn ein Druck in einer Umgebung des Sterilbehälters (10) einen Druck im Sterilbehälter (10) um eine vorgegebene Druckdifferenz übersteigt, und daß das Überdruckventil (54, 52, 80) die Filtereinheit (54) umfaßt.

27. Sterilbehälter nach einem der voranstehenden Ansprüche, **dadurch gekennzeichnet, daß** die Gasaustauschöffnung (26) mindestens ein Versteifungselement (30) zum Versteifen des Deckels (14) umfaßt.

28. Sterilbehälter nach Anspruch 27, **dadurch gekennzeichnet, daß** das mindestens eine Versteifungselement (30) durch einen die Gasaustauschöffnung (26) überdeckenden Steg gebildet wird.

29. Sterilbehälter nach einem der Ansprüche 27 oder 28, **dadurch gekennzeichnet, daß** das mindestens eine Versteifungselement (30) in Richtung vom Aufnahmeraum relativ zur Außenseite (37) des Deckels (14) weg versetzt ist.

30. Vorrichtung nach einem der voranstehenden Ansprüche, **dadurch gekennzeichnet, daß** ein Fluidrückhalteelement (130) zum Verhindern des Einströmens von Fluid von der Außenseite (37) des Deckels (14) durch die Gasaustauschöffnung (26) vorgesehen ist.

31. Vorrichtung nach Anspruch 30, **dadurch gekennzeichnet, daß** das Fluidrückhalteelement (130) auf der Außenseite (37) des Deckels (14) angeordnet ist und die Gasaustauschöffnung (26) mindestens abschnittsweise umgibt.

32. Vorrichtung nach einem der Ansprüche 30 oder 31, **dadurch gekennzeichnet, daß** das Fluidrückhalteelement als von der Außenseite des Deckels vorspringender Rand (130) ausgebildet ist.

33. Vorrichtung nach einem der Ansprüche 30 bis 32, **dadurch gekennzeichnet, daß** das Fluidrückhalteelement von der Gasaustauschöffnung (26) beabstandet angeordnet ist.

34. Vorrichtung nach einem der Ansprüche 30 bis 33, **dadurch gekennzeichnet, daß** das Schutzelement (98) die Gasaustauschöffnung (26) und das Fluidrückhalteelement vollständig überdeckt.

35. Sterilbehälter nach einem der voranstehenden Ansprüche, **dadurch gekennzeichnet, daß** der Deckel (14) aus einem Kunststoff hergestellt ist, insbesondere aus Polyetheretherketon (PEEK) oder Polyphenylensulfon (PPSU).

## Claims

1. Sterile container (10), in particular for the holding and sterile storage of surgical instruments or material, comprising a holding space, which is defined by a container base and container walls, and a lid (14), having a gas exchange opening (26) closed off by a sterile filter (62), for closing the holding space, wherein on an outer side (37) of the lid (14) there is provided at least one inflow edge (40) which faces away from the gas exchange opening (26) and slopes downward toward the outside rotative to a lid plane (15) and wherein there is provided a protective element (98) which covers the sterile filter (62) at a spacing therefrom, **characterized in that** there is provided at least one snap-action connection (120, 122) for connecting the protective element (98) to the lid (14).

2. Sterile container according to claim 1, **characterized in that** an angle of inclination (44) which is included by the inflow edge (40) and the lid plane (15) is at least 1 degree.

3. Sterile container according to one of the preceding claims, **characterized in that** the protective element (98) is disposed on the outer side (37) of the sterile container (10).

4. Sterile container according to one of the preceding claims, **characterized in that** the lid (14), on its outer side (37), has a seating surface (34) for seating the protective element (98), which surrounds the gas exchange opening (26).

5. Sterile container according to claim 4, **characterized in that** the seating surface comprises an annular surface (34) which runs parallel to the lid plane (15) and is set back in the direction of the holding space.

6. Sterile container according to either of claims 4 and 5, **characterized in that** the protective element (98) completely covers the gas exchange opening (26) and the seating surface (34).

7. Sterile container according to one of the preceding claims, **characterized in that** there are provided support surfaces (42) for the purpose of supporting the protective element (98) connected to the lid (14).

8. Sterile container according to one of the preceding claims, **characterized in that** the protective element (98) comprises spacing elements (112, 116) for maintaining a spacing between the protective element (98) and the lid (14).

9. Sterile container according to one of the preceding claims, **characterized in that** the protective element (98) comprises a reinforcing frame (108, 112).

10. Sterile container according to claim 9, **characterized in that** the reinforcing frame comprises webs (112) which cross one another and run substantially parallel to the lid plane (15).

11. Sterile container according to claim 10, **characterized in that** the spacing elements are formed by projections (116) and/or at least in part by the webs (112).

12. Sterile container according to one of the preceding claims, **characterized in that** the at least one snap-action connection (120, 122) is provided for connection of the protective element (98) to a rim (28), which includes latching recesses (32, 33), of the gas exchange opening (26).

13. Sterile container according to one of the preceding claims, **characterized in that** the at least one snap-action connection comprises elastic spring arms (120) which project away from the protective element (98) toward the lid (14) and are provided with a latching projection (122).

14. Sterile container according to one of the preceding claims, **characterized in that** there is provided, between the protective element (98) and the lid (14), at least one opening (124) for the passage of gas, which is in fluid communication with the gas exchange opening (26).

15. Sterile container according to claim 14, **characterized in that** the opening (124) for the passage of gas is arranged in such a way that it becomes possible for gas to flow in a direction of flow running substantially transversely with respect to the flow-permitting direction through the sterile filter (62).

16. Sterile container according to either of claims 14 and 15, **characterized in that** the opening (124) for the passage of gas, in cross section, has a double-convex lens shape.

17. Sterile container according to one of the preceding claims, **characterized in that** the lid (14) has at least one spacer element (46) for stacking a further sterile container on the sterile container (10), so that gas exchange through the gas exchange opening (26) is possible with stacked sterile containers (10).

18. Sterile container according to claim 17, **characterized in that** the inflow edge (40) is disposed between two spacer elements (46).

19. Sterile container according to either of claims 17 and 18, **characterized in that** the spacer elements comprise at least three projections (46) facing away from the outer side (37) of the sterile container (10).

20. Sterile container according to one of claims 17 to 19, **characterized in that** the seating surface (34) is delimited by the at least one inflow edge (40) and the at least one spacer element (46).

21. Sterile container according to one of claims 17 to 20, **characterized in that** the spacer elements (46) comprise or adjoin the support surfaces (42).

22. Sterile container according to one of claims 17 to 21, **characterized in that** the lid (14), the at least one spacer element (46), the seating surface (34) and the at least one inflow edge (40) are formed integrally.

23. Sterile container according to one of the preceding claims, **characterized in that** there is provided a filter unit (54), which comprises the sterile filter (62), a carrier (58) and a holding element (66), and **in that** the sterile filter (62) is held between the carrier (58) and the holding element (66).

24. Sterile container according to claim 23, **characterized in that** the filter unit (54) is in single-piece form, and **in that** the carrier (58), the sterile filter (62) and the holding element (66) are nonreleasably connected to one another.

25. Sterile container according to either of claims 23 and 24, **characterized in that** the filter unit (54) is mounted moveably, **in that** the filter unit (54), in a closed position, closes a flow path (126) and, in a flow-permitting position, opens the flow path (126), so that gas exchange in the closed position is possible only through the sterile filter (62) and in the flow-permitting position is possible through the sterile filter (62) and/or through the flow path (126).

26. Sterile container according to one of claims 23 to 25, **characterized in that** there is provided a pressure-relief valve (54, 52, 80), **in that** the pressure-relief valve (54, 52, 80) is disposed in such a way that in a basic position it adopts a closed position, that it adopts a flow-permitting position when a pressure in the vicinity of the sterile container (10) exceeds a pressure in the sterile container (10) by a predetermined pressure difference, and **in that** the pressure-relief valve (54, 52, 80) comprises the filter unit (54).

27. Sterile container according to one of the preceding claims, **characterized in that** the gas exchange opening (26) comprises at least one stiffening element (30) for stiffening the lid (14).

28. Sterile container according to claim 27, **characterized in that** the at least one stiffening element (30) is formed by a web which covers the gas exchange opening (26).

29. Sterile container according to either of claims 27 and 28, **characterized in that** the at least one reinforcing element (30) is offset in a direction away from the holding space relative to the outer side (37) of the lid (14).

30. Device according to one of the preceding claims, **characterized in that** there is provided a fluid-retaining element (130) for preventing fluid from flowing in from the outer side (37) of the lid (14) through the gas exchange opening (26).

31. Device according to claim 30, **characterized in that** the fluid-retaining element (130) is disposed on the outer side (37) of the lid (14) and surrounds the gas exchange opening (26) at least in sections.

32. Device according to either of claims 30 and 31, **characterized in that** the fluid-retaining element is formed as a rim (130) which protrudes from the outer side of the lid.

33. Device according to one of claims 30 to 32, **characterized in that** the fluid-retaining element is disposed at a spacing from the gas exchange opening (26).

34. Device according to one of claims 30 to 33, **characterized in that** the protective element (98) completely covers the gas exchange opening (26) and the fluid-retaining element.

35. Sterile container according to one of the preceding claims, **characterized in that** the lid (14) is made from a plastic, in particular from polyether ether ketone (PEEK) or polyphenylene sulfone (PPSU).

## Revendications

1. Récipient stérile (10), en particulier pour le logement et la conservation stérile d'instruments ou de matériels chirurgicaux, comprenant un compartiment de réception formé par un fond de récipient et des parois de récipient et un couvercle (14) pour la fermeture du compartiment de réception avec une ouverture d'échange gazeux (26) obturée par un filtre stérile (62), dans lequel il est prévu sur un côté extérieur (37) du couvercle (14) au moins une arête d'entrée (40) orientée à l'opposé de l'ouverture d'échange gazeux (26), retombant vers l'extérieur par rapport à un plan de couvercle (15), et dans lequel il est prévu un élément de protection (98) recouvrant le filtre stérile (62) avec un écartement, **caractérisé en ce qu'**il est prévu au moins un assemblage à enclenchement (120, 122) pour l'assemblage de l'élément de protection (98) avec le couvercle (14).

2. Récipient stérile selon la revendication 1, **caractérisé en ce qu'**un angle d'inclinaison (44) formé par l'arête d'entrée (40) et le plan de couvercle (15) est d'au moins 1 degré.

3. Récipient stérile selon l'une quelconque des revendications précédentes, **caractérisé en ce que** l'élément de protection (98) est placé sur le côté extérieur (37) du récipient stérile (10).

4. Récipient stérile selon l'une quelconque des revendications précédentes, **caractérisé en ce que** le couvercle (14) comporte sur son côté extérieur (37) une surface d'appui (34) pour la mise en place de l'élément de protection (98) qui entoure l'ouverture d'échange gazeux (26).

5. Récipient stérile selon la revendication 4, **caractérisé en ce que** la surface d'appui comprend une surface annulaire (34) s'étendant parallèlement au plan de couvercle (15), décalée vers l'arrière dans la direction du compartiment de réception.

6. Récipient stérile selon l'une quelconque des revendications 4 ou 5, **caractérisé en ce que** l'élément de protection (98) recouvre totalement l'ouverture d'échange gazeux (26) et la surface d'appui (34).

7. Récipient stérile selon l'une quelconque des revendications précédentes, **caractérisé en ce que** des surfaces portantes (42) sont prévues pour soutenir l'élément de protection (98) assemblé avec le couvercle (14).

8. Récipient stérile selon l'une quelconque des revendications précédentes, **caractérisé en ce que** l'élément de protection (98) comprend des éléments d'écartement (112, 116) pour maintenir un écart entre l'élément de protection (98) et le couvercle (14).

9. Récipient stérile selon l'une quelconque des revendications précédentes, **caractérisé en ce que** l'élément de protection (98) comprend un cadre de renforcement (108, 112).

10. Récipient stérile selon la revendication 9, **caractérisé en ce que** le cadre de renforcement comprend des nervures (112) qui se croisent et s'étendent sensiblement parallèlement au plan de couvercle (15) .

11. Récipient stérile selon la revendication 10, **caractérisé en ce que** les éléments d'écartement sont formés par des parties en saillie (116) et/ou au moins partiellement par les nervures (112).

12. Récipient stérile selon l'une quelconque des revendications précédentes, **caractérisé en ce que** le au moins un assemblage à enclenchement (120, 122) est prévu pour l'assemblage de l'élément de protection (98) avec un bord (28) de l'ouverture d'échange gazeux (26) comportant des entailles à crans (32, 33).

13. Récipient stérile selon l'une quelconque des revendications précédentes, **caractérisé en ce que** le au moins un assemblage à enclenchement comporte des bras à ressort (120) élastiques munis d'une partie en saillie à crans (122), s'écartant de l'élément de protection (98) dans la direction du couvercle (14).

14. Récipient stérile selon l'une quelconque des revendications précédentes, **caractérisé en ce qu'**il est prévu au moins une ouverture de passage de gaz (124) en communication fluidique avec l'ouverture d'échange gazeux (26) entre l'élément de protection (98) et le couvercle (14).

15. Récipient stérile selon la revendication 14, **caractérisé en ce que** l'ouverture de passage de gaz (124) est disposée de manière à permettre un écoulement de gaz dans un sens d'écoulement sensiblement perpendiculaire au sens passant du filtre stérile (62).

16. Récipient stérile selon l'une quelconque des revendications 14 ou 15, **caractérisé en ce que** l'ouverture de passage de gaz (124) présente une forme lenticulaire convexe double en section transversale.

17. Récipient stérile selon l'une quelconque des revendications précédentes, **caractérisé en ce que** le couvercle (14) comporte au moins un élément d'écartement (46) pour l'empilage d'un autre récipient stérile sur le récipient stérile (10), de manière à permettre un échange gazeux à travers l'ouverture d'échange gazeux (26) lorsque les récipients stériles (10) sont empilés.

18. Récipient stérile selon la revendication 17, **caractérisé en ce que** l'arête d'entrée (40) est placée entre deux éléments d'écartement (46).

19. Récipient stérile selon l'une quelconque des revendications 17 ou 18, **caractérisé en ce que** les éléments d'écartement comprennent au moins trois parties en saillie (46) orientées à l'opposé du côté extérieur (37) du récipient stérile (10).

20. Récipient stérile selon l'une quelconque des revendications 17 à 19, **caractérisé en ce que** la surface d'appui (34) est limitée par la au moins une arête d'entrée (40) et le au moins un élément d'écartement (46).

21. Récipient stérile selon l'une quelconque des revendications 17 à 20, **caractérisé en ce que** les éléments d'écartement (46) comprennent les surfaces portantes (42) ou sont adjacents à celles-ci.

22. Récipient stérile selon l'une quelconque des revendications 17 à 21, **caractérisé en ce que** le couvercle (14), le au moins un élément d'écartement (46), la surface d'appui (34) et la au moins une arête d'entrée (40) sont formés en une seule partie.

23. Récipient stérile selon l'une quelconque des revendications précédentes, **caractérisé en ce qu'**il est prévu une unité de filtrage (54) comprenant le filtre stérile (62), un support (58) et un élément de retenue (66) et **en ce que** le filtre stérile (62) est maintenu entre le support (58) et l'élément de retenue (66).

24. Récipient stérile selon la revendication 23, **caractérisé en ce que** l'unité de filtrage (54) est formée en une seule partie et **en ce que** le support (58), le filtre stérile (62) et l'élément de retenue (66) sont reliés les uns aux autres de manière inséparable.

25. Récipient stérile selon l'une quelconque des revendications 23 ou 24, **caractérisé en ce que** l'unité de filtrage (54) est logée de manière mobile, **en ce que** l'unité de filtrage (54) ferme une voie d'écoulement (126) dans une position de fermeture et ouvre la voie d'écoulement (126) dans une position de passage, de sorte que, dans la position de fermeture, un échange gazeux est rendu possible seulement par le filtre stérile (62) et, dans la position de passage, seulement par le filtre stérile (62) et/ou par la voie d'écoulement (126) .

26. Récipient stérile selon l'une quelconque des revendications 23 à 25, **caractérisé en ce qu'**il est prévu une soupape de surpression (54, 52, 80), **en ce que** la soupape de surpression (54, 52, 80) est placée de telle manière qu'elle prend une position de fermeture dans une position initiale, **en ce qu'**elle prend une position de passage lorsqu'une pression dans un environnement du récipient stérile (10) dépasse une pression dans le récipient stérile (10) équivalente à une pression différentielle prédéterminée et **en ce que** la soupape de surpression (54, 52, 80) comprend l'unité de filtrage (54).

27. Récipient stérile selon l'une quelconque des revendications précédentes, **caractérisé en ce que** l'ouverture d'échange gazeux (26) comprend au moins un élément raidisseur (30) pour raidir le couvercle (14).

28. Récipient stérile selon la revendication 27, **caractérisé en ce que** le au moins un élément raidisseur (30) est formé par une nervure recouvrant l'ouverture d'échange gazeux (26).

29. Récipient stérile selon l'une quelconque des revendications 27 ou 28, **caractérisé en ce que** le au moins un élément raidisseur (30) est décalé dans la direction du compartiment de réception par rapport au côté extérieur (37) de couvercle (14).

30. Récipient stérile selon l'une quelconque des revendications précédentes, **caractérisé en ce qu'**il est prévu un élément de retenue de fluide (130) pour empêcher la pénétration de fluide du côté extérieur (37) du couvercle (14) à travers l'ouverture d'échange gazeux (26).

31. Récipient stérile selon la revendication 30, **caractérisé en ce que** l'élément de retenue de fluide (130) est disposé du côté extérieur (37) du couvercle (14) et entoure l'ouverture d'échange gazeux (26) au moins par segments.

32. Récipient stérile selon l'une quelconque des revendications 30 ou 31, **caractérisé en ce que** l'élément de retenue de fluide est configuré comme un bord (130) faisant saillie à partir du côté extérieur du couvercle.

33. Récipient stérile selon l'une quelconque des revendications 30 à 32, **caractérisé en ce que** l'élément de retenue de fluide est placé à distance de l'ouverture d'échange gazeux (26).

34. Récipient stérile selon l'une quelconque des revendications 30 à 33, **caractérisé en ce que** l'élément de protection (98) recouvre totalement l'ouverture d'échange gazeux (26) et l'élément de retenue de fluide.

35. Récipient stérile selon l'une quelconque des revendications précédentes, **caractérisé en ce que** le couvercle (14) est fait en une matière synthétique, en particulier en polyétheréther-cétone (PEEC) ou en polyphénylène sulfone (PPSU).
